Europäisches Patentamt

European Patent Office

Office européen des brevets

(19)

(11) **EP 1 288 308 A1**

(12) **EUROPEAN PATENT APPLICATION**

(43) Date of publication:
05.03.2003 Bulletin 2003/10

(51) Int Cl.⁷: **C12Q 1/68**

(21) Application number: **01120466.6**

(22) Date of filing: **28.08.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicants:<br>• **Roche Diagnostics GmbH**<br>**68305 Mannheim (DE)**<br>Designated Contracting States:<br>**DE**<br>• **F. HOFFMANN-LA ROCHE AG**<br>**4070 Basel (CH)**<br>Designated Contracting States:<br>**AT BE CH CY DK ES FI FR GB GR IE IT LU MC NL PT SE TR LI** | (72) Inventors:<br>• **Weindel, Kurt, Dr.**<br>**82407 Wielenbach-Hardt (DE)**<br>• **Kraiss, Stefan, Dr.**<br>**80803 München (DE)**<br>• **Bergmann, Frank, Dr.**<br>**82393 Iffeldorf (DE)**<br>• **Josel, Hans-Peter, Dr.**<br>**82362 Weilheim (DE)**<br>• **Heindl, Dieter, Dr.**<br>**82327 Tutzing (DE)**<br><br>(74) Representative: **Knauer, Martin, Dr.**<br>**Roche Diagnostics GmbH,**<br>**Patent Department**<br>**68298 Mannheim (DE)** |

(54) **A method for the determination of multiple analytes**

(57) The present invention is directed to a method for the determination of at least 3 analytes using specific binding partners. Combinations of detectable labels are coupled to the binding partners assuring that each binding partner molecule is coupled with only one detectable label. This allows the determination of more different analytes than different labels used by not limiting the range of useable labels. The present invention is also related to kits, composition of matters and their use.

EP 1 288 308 A1

**Description**

This invention is directed to methods for the determination of multiple analytes using analyte specific binding partners wherein the binding partners are labeled with less different labels than different binding partners used. Additionally, this invention is directed to compositions of matter containing these differently labeled binding partners and the use of such compositions for the determination of multiple analytes as well as suitable kits.

**Background of the invention**

The determination of an analyte in a sample has aquired especial importance particularly in the field of health care, nutriation and ecology. Depending on the analyte different methods for determination can be used. Small molecules like metal ions, sugar monomers, amino acids often are determined by their chemical or physical properties. Analytes having a higher molecular weight like proteins and nucleic acid polymers can also be determined using binding partners having a specific affinity to them. Useful pairs of binding partners are antibody - antigen, substrate - enzyme, nucleic acid-complementary nucleic acid, sugar - lectin. In case it is intended to determine a specific protein for which one knows that it exhibits antigenic properties a specific antibody can be used for the determination. For the determination of a specific nucleic acid sequence a nucleic acid probe having a complementary sequence can be used. Such binding assays and suitable protocols are well known in the art and are fully explained in the literature, see for example Sambrook et al., 1985, Molecular Cloning - A Laboratory Manual, Coldspring Harbor Laboratory, Cold Spring Harbor, New York; Nucleic Acid Hybridization (B.D. Hames and S.J. Higgins, eds. 1984) and a series Methods in Enzymology (Academic Press, Inc.).

In order to allow the detection of the specific binding partner - analyte complexes it is very common to label the binding partners, whereby these labels can be detected directly or indirectly using additional reagents which bind to these labels (for example using biotin as a label, which can be detected using a avidin - horseraddich peroxidase conjugate and a suitable enzyme - substrate reaction for detection). Suitable labels are known in the art.

For many cases, especially in the diagnostic field it is necessary to determine two or more analytes in order to get a complete picture about a distinct situation. For example regarding a patient who might have a Chlamydia trachomatis infection, a doctor normally will not only check for a Chlamydia trachomatis but also for a Neisseria gonorrhoeae infection. Also when establishing the HLA pattern of a patient one need to determine the alleles of several different HLA loci.

When conducting a multiple analyte binding assay one has to pay attention to the labels used. In a first case the same label is used for all different binding partners. In this case two alternative formats have to be differentiated. If all binding reactions are conducted together in one reaction a positive result does only show that there is at least one analyte present in the sample, but not which one it is. As an alternative one can separate each binding reaction by conducting sequential reactions or using parallel reaction formats. Suitable formats are for example microtiterplate- or dot-blot-assays. Methods using such formats are known in the art. Such formats are time consuming and require a lot of handling steps, which could result in increased costs and contamination risks. More sophisticated methods using biochips are very expensive and are difficult to handle with regard to contaminations especially in the field of nucleic acid amplification.

In order to allow the discrete detection of several analytes within only one binding and/or detection reaction the different specific binding partners can be labeled using different labels, which can be separately detected. Such labels and useable detection methods are well known in the art. For example one can use labels which can be detected by its optical emission spectrum, whereby each of the label has a different emission spectrum. Although, it has to be noted that such assays are in reality limited to the determination of only a few analytes, because there is not an unlimited number of suitable labels which are detectable separately with high sensitivity, and sufficiently stable. In case of suitable rare earth labels, expensive and highly sophisticated detectors for time-resolved fluorimetry are necessary. These limitations are of special importance with regard to homogeneous detection methods. Such formats normally suffer from a higher signal background due to missing washing steps eliminating binding partners which are not bound to an analyte, which makes it difficult to interpret the multiple detection signals properly.

In order to increase the number of analytes detectable with a distinct number of labels also combinations of labels for labeling of binding partners can be used. In addition to binding partners having one distinct label attached also binding partners are used which are coupled with two or more labels using the same kind of labels also coupled to the single - labeled binding partners. Using for example 3 different labels 7 combinations of labels are possible by which also 7 different analytes can be detected. An in-situ-hybridization method using a similar principle is described by T. Ried et al., Proc. Natl. Acad. Sci USA, Vol. 89, pp. 1388- 1392 (1992). A similar method for determination of 7 different Human Papilloma Virus types in a sample using PCR and a subsequent probe hybridization assay are described by Samiotaki et al., Analytical Biochemistry Vol. 253, p. 156-161 (1997).

The labeling of binding partners with more than one label per binding partner molecule bears some disadvantages. Such multiple labeled binding partners are more difficult in synthesis and steric hindering of the labels can occur which might decrease the efficiency of the binding of the binding partner to ist analyte or the detection of the attached labels. Also interactions between the labels attached to the binding partner might occur, which could lead to wrong results. This could be relevant for example when using optical labels. It is certainly of especial importance when conducting homogeneous detection methods using fluorescence energy transfer labels as for example used in TaqMan assays (US 5210015, EP 0543942).

Therefore, it is an object of the present invention to improve the methods for determination of multiple analytes avoiding all or a part of the disadvantages of the known methods.

**Summary of the invention**

The main aspect of the present invention is related to a method for determination of multiple analytes using less labels than analytes determined. For this purpose combinations of labels are used. For example when determining 3 analytes only two different detectable labels are necessary. A first label is attached to a first binding partner specific for the first analyte. A second label is attached to a second binding partner specific for the second analyte. For labeling of the third binding partner specific for the third analyte the same kind of labels as has been coupled to the first and second binding partners are used. One amount of the third binding partner is coupled with the first label. In case of three analytes preferably one half of the third binding partner is labeled this way. Another part of the third binding partner is coupled to the second label, which would be in case of three analytes preferably the remaining half of the third binding partner. Therefore each binding partner specific for this analyte contains a first label or a second label. This results in binding partner molecules each labeled with only one label. In contrast thereto probes are described in the literature that have bound multiple labels to one probe molecule (Samiotaki, M. et al., Analytical Biochemistry 253, 156-161 (1997)). Such probes are more difficult to synthesize compared with the binding partners according to the present invention.

Different detection efficiencies which may occur for the different labels attached to the third binding partner, can be compensated for in 2 ways. In case a first label has a higher signal output compared to a second label, one can adjust this difference either chemically by mixing the third binding partner coupled to the first label with the third binding partner coupled to the second label in a defined non-1:1 ratio, or mathematically via normalization of signal output to a given label selected as standard. Such different detection efficiencies may be due to the intrinsic properties of the labels, such as absorptivity or quantum yield, but can also result from different coupling efficiencies, or susceptibility to solvent effects. In the latter case the mixing of the different amounts of the third binding partner following the coupling reaction provides a good possibility to adapt such discrepancies.

In addition possible interference which may occur when two or more detectable label are attached to one binding partner molecule is avoided. Therefore, binding partners according to the present invention are compatible with broad spectrum of labels, which also encompass fluorophores and combinations of fluorophores, like classical Förster type labels (Styer and Haugland, Proc. Natl. Acad. Sci. USA 98, 719 (1967)) which can be used for example in homogeneous detection assays like the TaqMan-method for determination of low concentrated nucleic acid analytes.

It should be noted that also an increased number of analytes may be determined using three or even more labels which can be combined in the same way as shown for the two labels. When using three labels up to seven analytes may be determined. Using four different labels even 15 analytes can be detected if applied singular as well as in dual, triplicate and quadruplicate combinations. If applied singular or in dual combinations only (which might be advantageous from a practical point of view), up to 10 different analytes can be differentiates with a set of 4 labels.

Therefore the present invention is related to a method for the determination of at least 3 analytes comprising the steps:

a) Providing a mixture of a sample containing said at least 3 analytes or suspected of containing one or more of said analytes and at least 3 different binding partners under conditions allowing the specific binding of said binding partners to said analytes, wherein

-    a first of said at least 3 binding partners is specific for a first of said at least 3 analytes and is coupled to a first label,

-    a second of said at least 3 binding partners is specific for a second of said at least 3 analytes and is coupled to a second label, which label is separately detectable from the label coupled to said first binding partner, and

-    a third of said at least 3 binding partners specific for a third of said at least 3 analytes whereby a first amount of said third binding partner is coupled to the same label as coupled to said first binding partner and a second amount of said third binding partner is coupled to the same label as the second binding partner and

said first amount of said third binding partner is not coupled to the same label as coupled to the second binding partner and said second amount of said third binding partner is not coupled to the same label as coupled to the first binding partner,

b) Detecting the signal intensities indicative for said first and second label,

c) Determining the analytes present in said sample using said signal intensities detected in step b).

Preferred analytes are nucleic acid analytes which can be determined using sequence specific probes. Such nucleic acid analytes can also be amplified nucleic acids using one of several nucleic acid amplification method known in the art, like LCR (U.S. Patent Nos. 5,185,243, 5,679,524 and 5,573,907; EP 0 320 308 BI; WO 90/01069; WO 89/12696; and WO 89/09835), cycling probe technology (U.S. Patent Nos. 5,011,769, 5,403,711, 5,660,988, and 4,876,187, and PCT published applications WO 95/05480, WO 95/1416, and WO 95/00667), Invader TM technology (U.S. Patent Nos. 5,846,717; 5,614, 402; 5,719,028; 5,541,311; and 5,843,669), Q-Beta replicase technology (U.S. Patent No. 4,786,600), NASBA (U.S. Patent No. 5,409,818; EP-0 329 822), TMA (U.S. Patent Nos. 5,399,491, 5,888,779, 5,705,365, 5,710,029), SDA (U.S. Patent Nos. 5, 455,166 and 5,130,238) and PCR (US-A-4,683,202), whereas the PCR method is most preferred.

The invention is also related to a composition of matter comprising

- a first binding partner specific for a first analyte coupled to a first label,

- a second binding partner specific for a second analyte coupled to a second label, which label is separately detectable from the label coupled to said first binding partner, and

- a third binding partner specific for a third analyte whereby a first amount of said third binding partner is coupled to the same label as coupled to said first binding partner and a second amount of said third binding partner is coupled to the same label as the second binding partner and

said first amount of said third binding partner is not coupled to the same label as coupled to the second binding partner and said second amount of said third binding partner is not coupled to the same label as coupled to the first binding partner.

and its use for the determination of at least three analytes in a sample.

Furthermore the invention is related to kits for determination of at least three analytes.

**Brief description of the drawings**

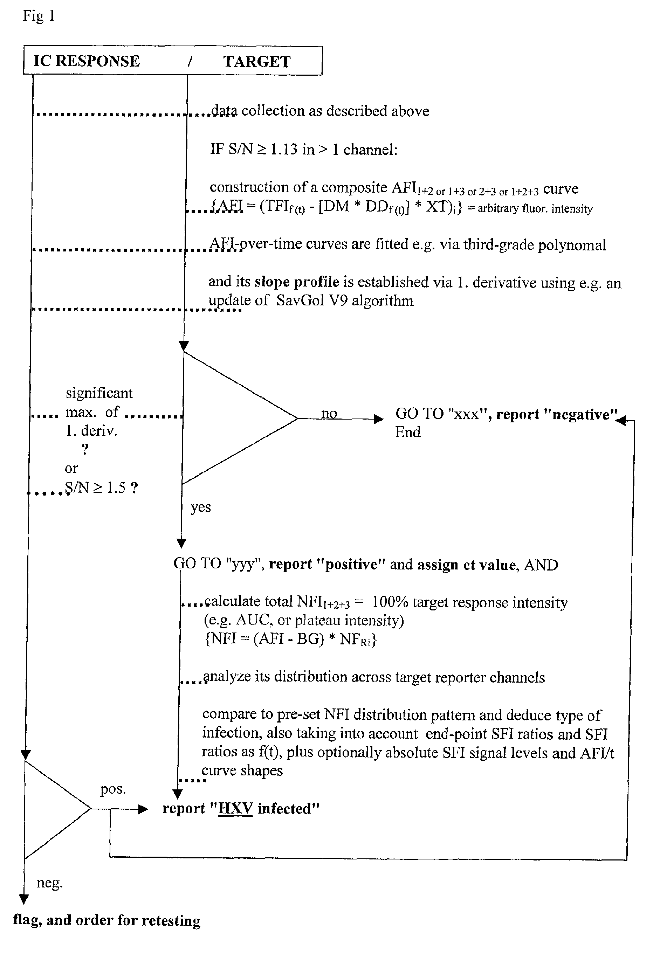

Fig. 1 shows a possible data processing scheme for an automated multiple analyte determination assay using the determination methods described. See also example 2.

**Detailed description of the invention**

Analytes according to the present invention are analytes which can be determined by binding assays known in the art. These are preferably components of samples for medical dignostics or other biological analytics, i.e. in particularly ingredients of body components such as antigens, antibodies, cells or nucleic acids. Such assays can be used for example for determination of infectious agents like bacteria e.g. chlamydia, neisseria and mycobacteria and viruses like HBV, HCV and HIV. Depending on the aim of an assay one can determine the amount of an analyte present in a sample as well as the configuration of an analyte, for example a nucleic acid analyte can be analysed for its allelic form or whether a patient carries a mutated form.

A sample according to the present invention contains the analyte to be determined. With regard to medical diagnostics samples derived from human or animal are preferred, e.g. whole blood, tissue sections, urine, sputum, serum, plasma, buffy coat and smears can be used. Depending on the analyte and the sample it might be necessary to pre-process the sample in order to allow determination of the analyte, for example for most samples nucleic acids need to be extracted in a first step. Such pre-processed samples are also samples according to the present invention.

According to the present invention at least three analytes are determined. This could be for example a pattern of infectious agents in a patient, like the viruses HIV, HBV and HCV, which have to be tested for each blood sample in blood banks. Another example could be the determination of the histocompatibility locus antigene pattern, which consists of several loci and distinct allelic forms.

The analytes are bound by specific binding partners. Depending on the analyte one has to choose specific binding partners. Analytes having antigenic properties can be bound by using specific antibodies. Antibodies in a sample can be determined by using the specific antigens as binding partners. If a nucleic acid analyte should be determined one can use a nucleic acid sequence being complementary to the analyte as specific probe. Further specific binding pairs like substrate - enzyme or sugar - lectin are known in the art, which might also be useful in the described methods.

A nucleic acid analyte is usually brought into available form by processing the original sample with one of various methods. This comprises for example change of pH (alkaline), heating, cyclic changes of temperature (freezing/thawing), change of the physiological growing conditions, use of detergents, chaotropic salts or enzymes (for example proteases or lipases), alone or in combination. For example one can use magnetic glass particles which are able to bind nucleic acid under specific conditions. Suitable particles and protocols are described in WO 96/41811 and WO 01/37291.

It is important to choose reaction conditions which promote the specific binding of the binding partners to the analytes which allows that the specific binding complexes occur but minimize the binding of the binding partners to unrelated reaction and sample components leading to an increased background signal. Such reaction conditions and suitable protocols are known in the art.

A specific nucleic acid binding partner, also called probe, for determination of a nucleic acid analyte or an amplified nucleic acid analyte preferably is an oligonucleotide, but also analogues having for example a peptide-backbone instead of the natural phosphate-sugar backbone (PNA, WO 92/20702) can be used. In order to allow a specific binding of the probe to the analyte the probes are preferably longer than 10 nucleotides, even more preferred have a length of 10 to 40 nucleotides. It is further required that the probe is sufficiently complementary to the analyte sequence. Therefore probes are preferably at least 80 % complementary, more preferred are at least 90 % complementary. In the most preferred case the probes are fully complementary to the analyte. The exact determination of complementarity and homology can be determined by using computer programs such as FastA (Pearson and Lipman, Proc. Natl. Acad. Sci. USA Vol. 85, pp. 2444- 2448 (88)).

Depending on the initial concentration of a nucleic acid analyte it might be necessary to amplify the analyte in order to allow its determination. For this purpose several amplification methods are known in the art (as referenced above). Especially when using primer based amplification methods a specific amplification of a nucleic acid analyte is possible. In combination with a probe binding assay an increased specificity of an assay can achieved. Also methods are known in the art, like LCR, which use labeled primers in order to allow the detection of an analyte. Also such methods can be modified according to the present invention.

A primer according to the present invention is a molecule capable of being extended or modified preferably by enzymes, more preferably by a polymerase of for instance procaryotic origin, when hybridized to a nucleic acid template. When using PCR methodology thermostable polymerases like *T. aquaticus* or *T. thermophilus* DNA-polymerase are preferred. These extend primers by adding mononucleotide units from monodesoxyribonucleosidetriphosphates to the 3'-OH-terminal end of said primers. The overall length and base sequence of a primer is dictated by the required specificity of the amplification reaction. Preferred primer lengths for performing PCR are from 10 to 40, most preferred from 15 to 30 base containing subunits, selected from mononucleotides and/or nucleic acid analog monomers. In general primers of that length are also useful for other amplification methods. If more than one primer is used for amplification, for example when using PCR or amplifying multiple target nucleic acids in one reaction, preferably primers are used which cannot hybridize to each other, because they do not contain any stretch of more than 5 consecutive complementary bases.

Labels are generally known to those skilled in the art as being a group which is detectable or can be made detectable for determination the presence of an analyte. Well-known labels are fluorescent labels, like fluoresceine and lanthanide chelates, electrochemiluminescent labels, like ruthenium complexes, or moieties that can be recognized by another molecular entity, like haptens which can be recognized by an antibody raised against this hapten or moieties that can be immobilized, like biotin which can be bound for example to streptavidin coated solid phases, like beads or tubes. Most preferred labels especially with regard to homogenous formats are chromophores. Such chromophores can be used alone or in combination with another chromophore or for example with a non-fluorescent quencher.

In case homogeneous detection formats are used, especially for determination of nucleic acid analytes several formats are known in the art. Method like the TaqMan assay (US Nos. 5,210,015 and 5,487,972) and the kissing probe-assay are based on fluorescent energy transfer of fluorescent dyes (FET, Styer and Haughland Proc. Natl. Acad. Sci. USA Vol. 98, pp. 719- 67). When brought in close proximity with each other a first fluorophor can interact with a second fluophor or a non-fluorescent quencher. For example, the electromagnetic emission or vibrational excitation of a first fluorescent dye can induce resonance in a second fluorescent dye. Depending on the format used one can for example detect a decreasing light emission of the first fluorescent dye or an increasing light emission of the second fluorescent dye as a mean for the presence of an analyte. Combinations of fluorescent dyes, which can be used for this purpose, are known in the art. Examples are classical dyes suitable for Förster-type Resonance Energy Transfer like Pentamethin-indodicarbocyanin (Cy5) combined with 6-Carboxyfluores-cein (6-FAM).

Label in this context means a signal generating entity, which is actually detected in the method of the present invention. With regard to the homogeneous formats described, pairs of fluorochromes are used, which get into resonance with each other and which are detected in these methods as a single detection signal. Although two distinct molecules, for example two fluorochromes, are involved, they function as one single label. In addition, the term label has to be understood that it could also mean that more than one label molecule of a distinct label is coupled to a binding partner molecule.

Depending on the labels used different detectors have to be used for measuring of the signals of the labels. Beside others, especially fluorimeters are widely used for this purpose. Fluorescent labelling is most advantageous in conjunction with homogeneous PCR, as no chemical trigger reagent has to be added for signal generation, in contrast to enzymatic or chemiluminescent labelling techniques. This allows for a closed tube procedure, the most effective way to avoid cross-contamination with amplified material. When determining multiple label signals it is important that the signals are distinguishable from each other. In order to avoid such cross-talk it is preferred when using optical labels that each label has a different emission and/ or extinction-spectrum. Although when using most of the commercially available labels in multiple analyte detection assay at least some cross-talk cannot be avoided. When determining only a few analytes this cross-talk can be compensated by processing the measured signals with computer programs or using more expensive spectral fluorimeters instead of filter based fluorimeters. With regard to homogenous formats especially when using fluorescence energy transfer-based methods the need of additional chromophores even increases the possibilities of interferences. Therefore in reality the number of suitable labels which can be used together in a multiple assay is limited.

According to the present invention at least one of the binding partners used is coupled with a combination of detectable labels, whereby each of these binding partner molecules is coupled to only one detectable label. In contrast probes as described by Samiotaki et al (as referenced above) are labeled with multiple labels (e.g. 10-20 coupled 5'-terminal per oligonucleotide). Due to the proximity of the detectable labels coupled to the same probe molecule the risk of interference between the labels is very high and not all types of labels especially fluorescent dyes can be used.

The methods according to the present invention can be used for determination of an increased number of analytes in a multiple analyte assay and are compatible with a broad range of labels.

By decreasing the number of labels necessary in a multiplex assay the need for exotic labels is avoided and also cheaper detectors like filter based fluorimeters can be used instead of spectral fluorimeters which are much more expensive. This further allows for homogeneous formats, which are of outstanding importance with regard to commercial diagnostic assays due to the limited contamination risks and fewer handling steps.

As indicated above, three labels combined in different combinations can be coupled with up to seven different specific binding partners allowing the distinct determination of six different analytes. This is visualized in the table below. P stands for Parameter = analyte, a channel corresponds to an optical tract as part of a detector optimised to capture the signal of a particular detectable label. The table shows the expected percentage distribution of signal over all three channels, being normalized either chemically or mathematically. Considered is the case that of the analytes P1 to P7 only one at a time would be present in a sample. The labeling of the different binding partners are: type P1 - Label1 (signal in channel 1, P2 - Label 2 (signal in channel 2), P3 - label 3(signal in channel 3), P4 - half amount label 1, half amount label 2, P5 - half amount label 1, half amount label 3, P6 - half amount label 2, half amount label 3 and P7 - 1/3 amount label 1, 1/3 amount label 2, 1/3 amount label 3.

| Parameter | Channel | | |
|---|---|---|---|
| | 1 | 2 | 3 |
| P1 | 100 | | |
| P2 | | 100 | |
| P3 | | | 100 |
| P4 | 50 | 50 | |
| P5 | 50 | | 50 |
| P6 | | 50 | 50 |
| P7 | 33 | 33 | 33 |

If a signal can be only measured in one channel a clear indication for the presence of one of the analytes of parameter 1, 2 or 3 is given (see table). If signals occur in two channels in a 50: 50 ratio there is a strong indication for an analyte of parameter 4, 5 or 6 as indicated in the table. A signal ratio of 33:33:33 would indicate that a P7 analyte is present in the sample. However this reflects an idealized situation. Real signals may deviate and often need to be normalized in order to compensate for example different detection efficiencies of the labels, background- and cross talk-signals.

The different labels coupled to the binding partners should not change the binding efficiency of a binding partner, which might also be adjustable by varying the ratios of binding partner bound to the first label: binding partner bound to the second label. Also different detection efficiencies can be adjusted by this way.

A 50: 50 signal ratio might also occur in case of the presence of two analytes. However this would probably be extremely rare, since their occurrence would depend on either a 1:1 co-infection in terms of number of starting molecules and identical extraction /amplification / detection efficiencies yielding a 50:50 signal distribution into 2 channels, or on different numbers of starting molecules in conjunction with extraction / amplification / detection efficiencies that precisely counter-balance the differences in titer and again yield a 50:50 distribution. Thus, co-infections of 2 parameters indicated by single labeled probe, respectively, e.g. P1 and P2, should result in an uneven distribution of >>50: <<50: 0 or <<50: >>50: 0 and so be distinguishable from e.g. P4 indicated by an even distribution of 50: 50: 0. However such results could also derived from samples co-infected with two agents for example P4 and P1.

In order to further decrease the risk of ambiguous results and possible errors in interpretation of the measured signals it is possible to arrange the parameters in a way that signals occurring in two or more channel can be clearly assigned to one possible analyte distribution in the sample as explained in example 1. In addition one can run an additional assay especially testing for the remaining possible analyte distributions which may be present in the sample. Such a sequential method is very economical as it allows to determine a distinct analyte distribution in most samples in a first run, whereas ambiguous samples can be re-evaluated in a second run using for example a multiple analyte assay with a reduced number of analytes (for which an unambiguous one label-one binding partner combination should be possible). Furthermore regarding nucleic acid analytes and amplified nucleic acid analytes one could for example determine the melting curve of the analyte-probe hybridization complexes or of the amplified nucleic acid, which allows distinguishing the different analytes and coming to an unambiguous result.

Also kits for determination of at least three analytes are subject of the present invention. Such kits containing in one or more containers

- a first binding partner specific for a first analyte coupled to a first label,

- a second binding partner specific for a second analyte coupled to a second label, which label is separately detectable from the label coupled to said first binding partner, and

- a third binding partner specific for a third analyte, whereby a first amount of said third binding partner is coupled to the same label as coupled to said first binding partner and a second amount of said third binding partner coupled to the same label as said second binding partner and
  said first amount of said third binding partner is not coupled to the same label as coupled to the second binding partner and said second amount of said third binding partner is not coupled to the same label as coupled to the first binding partner.

In addition such kits can also contain means for specific binding of the binding partners to their analytes, which may include buffers, blocking reagents and other reaction components known in the art. Kits for determination of nucleic acid analytes may also contain reaction components for nucleic acid amplification methods as described above. A PCR reaction kit could for example also contain a polymerase, buffers, nucleotide triphosphates and/ or primers.

The present invention is exemplified by the following examples:

## Examples

*Example 1*

Multi-color analysis according to the present invention can be done using the Roche Cobas Taqman amplification/ detection technology and 5'-nuclease assay technology (EP 0543942 and US 5210015) building on "classical" fluorochrome compounds and "classical" resonance energy transfer principles. A Roche Cobas Taqman instrument is equipped with up to 4 filter pairs (see also EP 0953379, EP 0953837, US 6134000 and US 6084669). A putative indicator set could consist of 4 reporters and preferentially 1 or 2, optionally non-fluorescent, quenchers. Candidate compounds that span the spectral range set by the Cobas Taqman detector, i.e. ca. 420 nm - ca. 710 nm, may include:

- for quencher (Q) dyes with broad-banded absorption range in the red, for instance polymethin-cyanine dyes (e.g. n = 5, i.e. 4 conjugated olefinic entities building the mesomeric structure), or rhodamin derivatives at the flanks symmetrically fused to unsaturated N-heterocyclyc moieties that participate in the conjugated mesomeric $\pi$-e$^{(-)}$-

system, both preferentially as non-fluorescent nitrophenyl-derivatives

- for reporter (R) dyes fluorescent molecules like coumarine dyes, fluorescein-type dyes like FAM or chlorinated derivatives, rhodamin-type dyes, oxazines or bodipy-type compounds. Three of these would be assigned to target parameters, one for monitoring IC.

General requirements pertinent to candidate dyes include: good chemical stability in stock solution and also at use level as ingredients in mastermix reagents; stable when exposed to ambient light during synthesis, purification and kit manufacture; stable when exposed to multiple heating /cooling cycles (e.g. n = 60) in PCR; sufficient spectral overlap and efficient energy transfer between corresponding RQ resonance pairs; high extinction coefficients (i.e. absorptivity); and especially for reporters high quantum yield in aquous solutions and in the pH range employed for Taqman-PCR (e.g. pH 7-8,5).

The new concept allows the measuring of the distribution of crosstalk- and background-corrected normalized signals of 3 reporter dyes and dual combinations thereof across the corresponding 3 optical channels of Cobas Taqman instead of having a fixed assignment of a particular dye, representing a particular assay parameter, and a corresponding channel. The principle would provide for a complete resolution (via reporter R1, R2, R3, R1+2, R1+3, R2+3) of a positive result obtained with a 6-parameter-multiplex assay, the maximum complexity taken into consideration.

Theoretically, in cases of single-label use, 100% of the respective processed signal is found in a given channel; in cases of dual label use, processed signals would be distributed evenly across 2 corresponding channels (50% + 50%).

Discrimination of positive / negative pools (donations) could be done based on an optimized algorithm for determination of threshold cycles (ct values, i.e. the point on the reaction coordinate, where the fluorescent signal intensity rises significantly above background level), sensing the slope profile of signal-over-time curves.

Resolution of the kind of infection (assay parameter, i.e. kind of pathogen, yielding detectable product) could be done via analysis of channel-specific contributions to the total area under the signal/time curves (AUC), or to the total normalized plateau signal intensity. There is no firm dose-response correlation for criteria like these which, however, is not mandatory for qualitative assays as needed for blood screening.

A suitable 6-parameter (P1 to P6) multiplex assay could comprise the following probes (Q= quencher, R= reporter and $N_i$= one of the four nucleotides):

parameter 1 - probe = 5'-Q-$(N_i)_g$-**R1**-( $N_i)_h$-3'-PO$_4$ + 5'-Q-$(N_i)_g$-**R2**-( $N_i)_h$-3'-PO$_4$ <u>5 pmol each</u> / 100 µl reaction mix
parameter 2 - probe = 5'-Q-$(N_i)_a$-**R1**-( $N_i)_b$-3'-PO$_4$ <u>10pmol</u> / 100 µl reaction mix
parameter 3 - probe = 5'-Q-$(N_i)_c$-**R2**-( $N_i)_d$-3'-PO$_4$ <u>10pmol</u> / 100 µl reaction mix
parameter 4 - probe = 5'-Q-$(N_i)_e$-**R3**-( $N_i)_f$-3'-PO$_4$ <u>10pmol</u> / 100 µl reaction mix
parameter 5 - probe = 5'-Q-$(N_i)_k$-**R1**-( $N_i)_l$-3'-PO$_4$ + 5'-Q-$(N_i)_k$-**R3**-( $N_i)_l$-3'-PO$_4$ <u>5 pmol each</u> / 100 µl reaction mix
parameter 6 - probe = 5'-Q-$(N_i)_n$-**R2**-( $N_i)_m$-3'-PO$_4$ + 5'-Q-$(N_i)_n$-**R3**-( $N_i)_m$-3'-PO$_4$ <u>5 pmol each</u> / 100 µl reaction mix

In other words:

in cases 2 - 4, ca. $1*6^{12}$ molecules of uniformly labelled probe (same oligonucleotide /same reporter) would be introduced into PCR per parameter;

in cases 1, 5 and 6, again ca.$1*6^{12}$ molecules of labeled probe would be introduced into PCR per parameter, ca. $\frac{1}{2}*6^{12}$ molecules with one sort of tag, and $\frac{1}{2}*6^{12}$ molecules with the other sort of tag (same oligonucleotide / different reporters), which would be consumed statistically balanced;

in all case, 10 pmol of parameter-specific probe would be available.

Regarding the risk of ambiguous results, and focusing on the use of singular labels and dual combinations only, one may argue that ambiguous results would probably be extremely rare in the case of co-infections of 2 parameters indicated by single labeled probe, respectively, e.g. P2 ⇔ R1 + P4 ⇔ R3. The probability of either a 1:1 co-infection in terms of number of starting molecules and identical extraction /amplification / detection efficiencies yielding a 50:50 signal distribution into 2 channels, or of different numbers of starting molecules in conjunction with extraction /amplification / detection efficiencies that precisely counter-balance the differences in titre and again yield a 50:50 distribution, is considered negligibly low. Thus, an uneven distribution of >(>)50 : <(<)50 : 0 or <(<)50 : >(>)50 : 0 would result (*case I*), and so be distinguishable from e.g. P5 ⇔ R1R3 indicated by an even distribution of 50 : 50 : 0.

Co-infection by 2 parameters indicated by 1:1 mixtures of 2 dual labeled probes *(case II)*, e.g. P1 ⇔ R1R2 and P5 ⇔ R1R3, should always be evident due to signal distribution into 3 channels (e.g. ca. 50 : 25 : 25, comparable extraction / amplification / detection eficiencies assumed).

Co-infection by 2 parameters, one represented by 1 reporter and the other by two, should be recognized due to signal distribution into 3 channels, ≈ 33 : 33 : *33* (***case III,*** e.g. R1R2 + R3), or yield a ≈75 : ≈25 : 0 pattern (***case IV,*** e.g. R1R2 + R1), again assuming comparable extraction / amplification / detection efficiencies. For case II and III, the common feature would that there is no optical channel related to target with essentially "zero signal". In other words, the "zero+x" threshold to discriminate against "true zero" will be important. In *case IV,* probably the most critical one, resolution does depend on the overall efficiency of the assay, which determines whether the resulting distribution is substanbtially different from a 50 : 50 situation, or not. Most probably, additional mathematical criteria have to be taken into view in oder to resolve ambiguities, especially of the latter kind. Advantageously, real-time Taqman-PCR offers such "helper arithmetics". This topic is dealt with below in some more detail.

The rare occurrences of ambiguity, i.e. the event of a 50 : 50 signal - distribution occuring from co-infection can further minimized by assigning extreme rarely found, and less efficiently amplified parameters to single labeled probes, which might be complemented by considering geographical distributions.

A putative assignment of reporters (R = Label) and assays for the determination of HIV-1-M, HIV-1-O, HIV-2, HCV, HBV and HAV could be as follows:

| P1 | ⇔ | HIV-1-M | ⇔ | R1/R2 | Amplification and | high |
| P2 | ⇔ | HIV-1-O | ⇔ | R1 | detection efficiency: | low |
| P3 | ⇔ | HIV-2 | ⇔ | R2 | | low |
| P4 | ⇔ | HCV | ⇔ | R3 | | high |
| P5 | ⇔ | HBV | ⇔ | R1/R3 | | high |
| P6 | ⇔ | HAV | ⇔ | R2/R3 | | medium |

IC (internal control) ⇔ R4.

For safety reasons and especially with regard to a commercial assay one can also add an internal control, which for example may be detected by a probe coupled to a fourth label (R4).

Here, co-infection of HIV-1-O and HIV-2 should be very unlikely, both being very rare and geographically well separated (which, however, may change over the years), and the overall result would be "HIV positive" even in case of such an unexpected event.

On the other hand, co-infections of HIV-1-O and HCV or HIV-2 and HCV should not result in an even distribution of 50 : 0 : 50 or 0 : 50 : 50, given the current amplification efficiencies (low for HIV-1-O and HIV-2), and not be mistaken for HBV or HAV single infections, respectively. Thus it is possible to use a standard four channel fluorimeter for conducting a six-parameter assay including an internal control being separately detected, which is for example used in a Roche Cobas TaqMan-instrument.

Thus, this concept for complete resolution of a 6 parameter multiplex assay in terms of assay parameter would necessitate moderate additional efforts compared to the present chemical system and instrument platform. Likewise, the requirements in terms of a more sophisticated analytical software are not very demanding, as shown by the considerations depicted below. In some detail, this will include:

- double logistic tracking for 3 probes (same oligonucleotide, different fluorescent tags)

- standardized procedure to establish signal outputs for individual reporters from single tagged probes and elaboration of normalization factors (taking into account impact from the sequence environment in the respective oligonucleotides, degree of purification as indicated by e.g. Em(R/Q) ratios, wavelength dependent energy contents and spectral sensitivity of CTM ASICS)

- elaboration of the most reliable output criterion or set of criteria to serve as 100% count, determination of the variance of distribution patterns, and setting of acceptance range borders for expected values

- development of a software algorithm for analysis of distribution of corrected normalized signals into 3 target channels that incorporates all the parameters mentioned above

*Example 2*

With regard to an assay protocol for automated test devices like the Roche Cobas TaqMan™ instrument a suitable process scheme is given below (see also Figure 1).

The availability of new reagents given, the principal data collecting and data processing scheme of an automated assay could be as follows:

| - | data collection: | | status: |
|---|---|---|---|
| 1. | dark mean (= instrument noise) | DM | already done |
| 2. | dark drift (= drift correction factor as f(t)) | DD | already done |
| 3. | cross-talk and preset correction factors | XT | already done |
| 4. | signal output normalization factors for reporters (= preset factors to correct for quantum yield, micro-environmental effects, spectral sensitivity, ...) | $NF_{Ri}$ | additional |
| 5. | background intensity (= chemical noise, accumulated from for all probes contained in the mix) | BG | already done |
| 6. | measured total signal over time | TFI | already done |

Preferably all steps are done separately for the optical channels 1, 2, 3, and 4 on the Cobas Taqman- instrument.

- data processing:

1) for each channel, and as a signal-over-time graph,
$\{TFI_{f(t)} - \{(DM * DD_{f(t)}) + BG]\} * XT * NF_{Ri} = \underline{NFI}$ is caluclated, i.e.
channel-specific normalized corrected fluorescence intensities
2) if channels 1, 2, 3 correspond to reporter dyes, and channel 4 to IC dye, then
$NFI_4$ = 100% IC response intensity
$NFI_{1+2+3}$ = 100% target response intensity,
3) which is to be analyzed in terms of distribution across these channels, i.e. x % in channel 1, y % in channel 2, and z % in channel 3.
The resulting signal distribution numbers are then compared to the pre-calculated values given above, and the identity of the pathogen detected is deduced from the correlation of optical channel-specific gain and corresponding reporter dyes, plus auxiliary factors.

Thus, in other words, the assay is not calibrated with respect to target titre (i.e. it is still a qualitative screening assay, yielding a pos/neg result as appropriate e.g. for screening applications in blood banks), but with respect to signal output of parameter-specific probes at a given concentration (to identify the cause for the positive result of a multiplex amplification).

The individual sample analysis could proceed as indicated in Figure 1.

To comprehend the logic of this processing algorithm, let's consider the underlying assay principles.

In 5'-nuclease technology (Taqman-PCR, the preferred embodiment for the invention set forth here) amplification and detection reactions, respectively, are closely interwoven. To this end, detection probes with 2 particular chemical modification are added to the PCR mastermix. One of these modifications is a fluorogenic reporter group (R, for instance a derivative of 6-carboxy-fluorescein) covalently attached to the backbone of the probe, the other one is a dye (for instance a polymethine-cyanine derivative) capable of absorbing the fluorescent light of the reporter and to quench it (quencher, Q). The quencher is typically attached to the probe backbone at the 5'-end, whereas the reporter is located within the oligo sequence, spaced from the quencher by a number of nucleotide building blocks. Probes bind to target nucleic acids (sense or anti-sense strand) close to the 3'-end of a primer (reverse or forward). As soon as primer has annealed to the target and DNA-polymerase gets bound to the primer :

target hybrid, elongation starts. Due to the 5'-nuclease activity of the enzyme, simultaneously with copy strand synthesis the probe is cleaved as soon as the polymerase reaches the probe binding site, reporter and quencher get separated, and the fluorescent signal becomes measurable. This process is repeated with every cycle, and more and more fluorescent reporter is accumulated in solution until reagent depletion at the end of the reaction. So, in a signal-over-time plot, sigmoidal growth curves are generated. The point on the time axis, where the arbitrary fluorescent intensity (AFI, also called relative light units, RLU) can be significantly distinguished from background signal is called threshold cycle (ct). ct is a measure of analyte titre: the smaller the ct-value, the higher the number of starting molecules and/or the better the extraction or amplification/detection efficiency. ct values can be calculated by means of different mathematical operations. For example, cut-off approaches (average background signal intensity multiplied by a constant factor yields a cut-off signal intensity to discern negative from positive) can be used, or approaches where the location of the maximum of the first or second derivative of the signal-over-time curve (i.e. the steepness or slope profile) is sensed after curve fitting. The slope profile approach is, in contrast to the signal threshold approach, essentially independent of the background intensity level and, thus highly attractive for multiplex assays with cumulated background from all probes present in the reaction mixture.

Based on these principles, and according to the processing scheme depicted above, one would employ different means to generate the primary result (i.e. positive or negative?), and the secondary result (i.e. which kind of infection renders the sample positive?).

Compared to performing a multiplex assay with one common label, there is potential loss in sensitivity due to dilution of signal into several optical channels and, thus, reduced specific signal growth curves in particular channels. Therefore, in order to generate the primary result, one would optionally work with composite signal-over-time curves. By this means, the entire specific signal yield is collected into one signal-over-time curve which then is used to determine the threshold cycle (ct) and the principal status of the sample (pos. / neg.). Using the slope profile approach, the addition of background intensity would not be detrimental, and one could exploit the potential advantage of sensing the more pronounced growth characteristics of the specific signal gain.

On the other hand, for generation of secondary results, one would refer to the channel-specific distribution of normalized signal (NFI intensities), and deduce the kind of infection by comparing the resulting distribution patterns to the pre-calculated matrix of patterns given above. In addition, and in order to enhance the discriminatory power, also non-normalized *specific* signal intensities may be considered for analysis of ca. 50 : ca. 50 or ca. 75 : ca. 25 distributions of normalized signal, i.e. the really critical ones.

**[0070]** - In case of a ***mono-infection*** represented by a 1:1 mixture of probes, the resulting ratio of non-normalized, yet background-corrected signals R1 / R2 would be constant throughout the entire amplification/detection process, for instance 40 / 50. This must be precisely equivalent to the value of the normalization factor (0,80) obtained with equal amounts of purified dye, because both sorts of labelled probe (same oligonucleotide sequence!) would be cleaved at identical rates. Background (BG)-correction is necessary, since with a resolving 6-parameter-multiplex assay according to the concept presented here, each channel-specific background is made up from the signal output of 3 different probes tagged with the same label. Therefore, BG intensity is not only a function of the optical features of the label, but also of the structural features of the probe, i.e. linear or hairpin, or (more generally) by the degree of self-complementarity, and of the temperature during measurements which affects conformational equilibria of secondary structures.. So, secondary structures of unhybridized probe must be taken into view with respect to BG intensity, and auxiliary calculations have to be based on *specific* fluorescence intensities, SFI, with

$$\text{SFI} = \text{TFI}_{f(t)} - [(\text{DM} * \text{DD}_{f(t)}) + \text{BG}] * \text{XT}_i = \text{AFI} - \text{BG}.$$

*Specific* signal, by contrast, is generated by way of 5'-nuclease activity of the polymerase depending on essentially perfect hybridization of probe to target, i.e. linearization of the probe oligonucleotide and, thus, essentially independent of probe structure. This pertains also to the truncated probe after having been cleaved and dissociated from the target strand due to the concomitant drop in Tm (melting temperature). Short internal hybrids in the residual probe oligomer should not be stable at the elevated temperatures used for annealing / elongation in PCR. After normalization, which compensates for differences in quantum yield or susceptibility to solvent effects, signal distribution in channel 1 and 2 would be 50 : 50. Finding NFI <u>and</u> SFI ratios as expected is double proof for mono-infection.

- In case of ***a double infection*** indicated via 2 specific probes labelled R1 and R2 (***case I***), respectively, it is very likely that the signal dynamics (i.e. growth as a function of time) is different for the 2 different assays, as either initial analyte titre, or overall assay efficiency, or both will probably be different. Thus, in addition to the probability of a > 50 : < 50 situation on the NFI level, also the SFI ratio of R1 / R2 must be different for rather early, central, and late phases of the reaction, i.e. the SFI ratio will be variable. The ratios will have a certain trend, and be different from the respective normalization factor obtained with equivalent mixtures of purified dye, preferably coupled to a model probe (to account for inevitable microenvironmental factors, e.g. linker, local charges), e.g. a $T_3$-R-$T_{16}$-oligonucleotide, which does not modulate signal output by its structural configuration.

- In case of a ***double infection*** represented by R1 + R1R2 (***case IV***) and substantially different overall recoveries for the 2 assays, a normalized signal distribution not very different from 50 : 50, e.g. 55 : 45, might be observed in channel 1 and 2. In terms of non-normalized signal intensities, however, the ratio R1 / R2 would be e.g. 44 / 50 (0,88), i.e. greater than the normalization factor. Thus, the double bias of "greater than expected" on both the normalized and non-normalized level, would indicate double infection. What is more, the ratio R1 / R2, e.g. traced e.g. via AUC, would most probably not be constant throughout the entire assay due to differing signal dynamics resulting from different amplification efficiencies, which will enhance discrimination.

- If, in case of double infection, both assays have ca. equal efficiency and start from either substantially different or ca. equal analyte titres, a 75 : 25 : 0 NFI pattern would be approximated, or even exceeded, with either a R1 + R2 or a R1 + R1R2 labelling (**case I OR case IV**?). Here, discrimination between a *case IV* and a strongly *biased case I*-type co-infection may be achieved by means of absolute *specific* signal intensities (R1 intensity should be much lower in a *case I* situation) or absolute differences of channel-specific, optionally rectified, SFI values. Results might not completely unambiguous in some cases, yet.

In addition to this, composite AFI/t curves may be analysed concerning curve shape characteristics. In case of a mono-infection, one does expect a mono-sigmoidal curve with 1 point of inflection. In case of a double infection, however, it is most likely that the 2 growth curves generated from each of the 2 kind of nucleic acid targets extracted from the 2 infectious agents are not identical in terms of ct (i.e. point on the time axis where the curve bends upward and rises above background and drift level), in terms of shape, and specific signal intensity. Thus, a bi-sigmoidal curve shape may be generated, with 2 points of inflection. So, mono- or bi-sigmoidal curve shapes might be indicative of mono- or bi-infections present in the sample under inspection. In unfavourable cases, however, superposition of 2 or more curves may lead to irregular shapes with no clearcut maximum of slope, and with noise (i.e. the roughness of the curve) being increased. Still another possibility is a composite curve with 1 point of inflection, when 2 nearly identical curves are superposed.

To sum up, there are a number of mathematical means available to achieve resolution of a positive multiplex assay result in terms of the type in infection. These include, but are not limited to:

Basic means - reference NFI distribution patterns, and
type of deviation, either in situations with significant signal intensity in 2, or in 3, optical channels related to target. The accepted range of deviations from the reference pattern can be set by absolute numbers, % bands relative to the respective NFI value, based on precision data, or a combination of these.

Auxiliary means - analysis of NFI and SFI ratios pertinent to particular optical channels for common bias;
analysis of NFI or SFI ratios as a function of cycle number , i.e. dynamically along the reaction coordinate (constant or variable);
relationship of NFI ratios and corresponding absolute SFI values, or absolute SFI differences between any 2 optical channels (optionally rectified by relating to a standard dose difference), taking into account known assay-specific overall efficiencies (extraction / amplification / detection) for the different infectious agents under inspection;
analysis of composite AFI-over-time curve shapes (if applicable)

What is more, in order to monitor the whole process, an artificial nucleic acid construct may be added to all samples, preferably packaged (armoured) in a modified virus particle, which is co-extracted and co-amplified with the natural target. This internal control (IC) features a unique probe binding region for an IC detection probe, which differs from target-specific probes by a different reporter group with distinguishable emission characteristics. Thus, IC signal can be discerned from target signal, and as the IC is known to be present in the sample, it functions as a monitoring agent. If there is no IC response, the respective reaction has to be considered invalid, and repeated.

Additional to the co-extracted/-amplified/-detected internal control (IC), added to each and every specimen to be processed (i.e. both unknowns and external controls or calibrators) and indicated by R4, external positive controls will be part of the assay. These are "knowns" which must be found positive, as opposed to "unknowns", and may be designed as mixed control samples that give rise to signal in all target related optical channels. Combined with the IC, utility of the entire optical system would be monitored then in addition to the functionality of the complete extraction/ amplification/detection process. For nucleic acid-based screening applications in blood banks, for instance, a highly sensitive, yet qualitative assay is required to identify each and every positive donation, as far as technically feasible. Therefore, titres of external postitive controls would be set in the lower range of concentration

*Example 3*

Based on the parameter list given in example 1 the signal distribution shown below is analyzed. For each parameter the signal distribution in Channel 1 (reporter R1), Channel 2 (reporter R2) and Channel 3 (reporter R3) is given by 6 parallel simulated data sets based on 6-fold determinations each, as shown on the table (right side). On the left side the subsequent steps for evaluating the signal data are shown.

Each reference pattern is complemented by a concomitant accepted range, for instance

| | Ch1 | Ch2 | Ch3 |
|---|---|---|---|
| P1 | *50* | *50* | 0 |
| P2 | *100* | 0 | 0 |
| P3 | 0 | *100* | 0 |
| P4 | 0 | 0 | *100* |
| P5 | *50* | 0 | *50* |
| P6 | 0 | *50* | *50* |
| NFI reference pattern | | | |

| | Ch1 | Ch2 | Ch3 |
|---|---|---|---|
| P1 | 10 | 10 | 9,17 |
| P2 | 10 | 1,67 | 6,25 |
| P3 | 7,08 | 10 | 7,92 |
| P4 | 4,17 | 1,67 | 10 |
| P5 | 10 | 4,17 | 10 |
| P6 | 5,83 | 10 | 10 |

Putative 10% / 20% off-set bands pertinent to 100% and 50% mean values, and 5 X multiples of near zero values, which translates into the following accepted ranges for the reference pattern

| | Ch1 | Ch2 | Ch3 |
|---|---|---|---|
| P1 | 40-60 | 40-60 | 0-9 |
| P2 | 90-100 | 0-2 | 0-6 |
| P3 | 0-7 | 90-100 | 0-8 |
| P4 | 0-4 | 0-2 | 90-100 |
| P5 | 40-60 | 0-8 | 40-60 |
| P6 | 0-8 | 40-60 | 40-60 |

Accepted ranges of deviation may be calculated, for example, as percent values (%), absolute numbers, multiples of simple standard deviation, or combinations thereof.

## Simulated distribution

|      | Ch1    | Ch2    | Ch3    |
|------|--------|--------|--------|
| P1   | *49,33* | *48,83* | 1,83   |
| P2   | *98,42* | 0,33   | 1,25   |
| P3   | 1,42   | *97,00* | 1,58   |
| P4   | 0,83   | 0,33   | *98,83* |
| P5   | *48,17* | 1,67   | *50,17* |
| P6   | 1,17   | *50,33* | *48,50* |

mean values (arithmetic)
*According to reference pattern*, <u>deviated</u>

|      | Ch1   | Ch2   | Ch3  |
|------|-------|-------|------|
| P1   | *5,25* | *6,12* | 5,17 |
| P2   | *4,69* | 2,45  | 3,25 |
| P3   | 2,75  | *5,02* | 3,34 |
| P4   | 3,51  | 1,55  | *4,81* |
| P5   | *7,92* | 6,75  | *7,92* |
| P6   | 3,51  | *6,48* | *7,29* |

threefold standard deviation
*according to reference pattern*, <u>deviated</u>

|      | Ch1  | Ch2  | Ch3 |
|------|------|------|-----|
| P1   | *50*  | *50*  | 0   |
| P2   | *100* | 0    | 0   |
| P3   | 0    | *100* | 0   |
| P4   | 0    | 0    | *100* |
| P5   | *50*  | 0    | *50*  |
| P6   | 0    | *50*  | *50*  |

*NFI reference pattern*

|      | Ch1   | Ch2   | Ch3  |
|------|-------|-------|------|
| P1   | 10,00 | 10,00 | 9,17 |
| P2   | 10,00 | 1,67  | 6,25 |
| P3   | 7,08  | 10,00 | 7,92 |
| P4   | 4,17  | 1,67  | 10,00 |
| P5   | 10,00 | 8,33  | 10,00 |
| P6   | 7,50  | 10,00 | 10,00 |

**10% / 20%-off-set bands pertinent to 100% and 50%-mean values, and factor 5 pertinent to 0% values i.e. accepted ranges for reference pattern**

## Simulated data-set:

|     | Ch1  | Ch2 | Ch3 | Total |
|-----|------|-----|-----|-------|
| P1  | 52   | 48  | 0   | 100   |
|     | 49   | 48  | 3   | 100   |
|     | 47   | 52  | 1   | 100   |
|     | 50   | 46  | 4   | 100   |
|     | 48   | 49  | 3   | 100   |
|     | 50   | 50  | 0   | 100   |
| P2  | 98   | 0   | 2   | 100   |
|     | 97,5 | 0   | 2,5 | 100   |
|     | 99   | 0   | 1   | 100   |
|     | 100  | 0   | 0   | 100   |
|     | 96   | 2   | 2   | 100   |
|     | 100  | 0   | 0   | 100   |
| P3  | 1    | 96  | 3   | 100   |
|     | 2    | 96  | 2   | 100   |
|     | 2    | 97  | 1   | 100   |
|     | 1    | 99  | 0   | 100   |
|     | 0    | 99  | 0   | 100   |
|     | 2,5  | 95  | 2,5 | 100   |
| P4  | 0    | 0   | 100 | 100   |
|     | 0    | 0   | 100 | 100   |
|     | 3    | 1   | 96  | 100   |
|     | 1    | 0   | 99  | 100   |
|     | 0    | 0   | 100 | 100   |
|     | 1    | 1   | 98  | 100   |
| P5  | 46   | 6   | 48  | 100   |
|     | 50   | 1   | 49  | 100   |
|     | 45   | 0   | 55  | 100   |
|     | 52   | 0   | 48  | 100   |
|     | 49   | 1   | 50  | 100   |
|     | 47   | 2   | 51  | 100   |
| P6  | 0    | 50  | 50  | 100   |
|     | 1    | 49  | 50  | 100   |
|     | 1    | 53  | 46  | 100   |
|     | 2    | 47  | 51  | 100   |
|     | 3    | 52  | 45  | 100   |
|     | 0    | 51  | 49  | 100   |

With regard to P5 a distribution of Ch1:Ch2:Ch3 of approximately 50:0:50 is found, with both mean values and scatter being within the accepted ranges. This would lead to the result "P5-positiv", which means single infection with HBV.

*Example 4*

Data set as in Example 1, execept for P5, i.e. signal distribution in channels 1 and 3.

## Simulated distribution

|    | Ch1 | Ch2 | Ch3 |
|----|------|------|------|
| P1 | *49,33* | *48,83* | 1,83 |
| P2 | *98,42* | 0,33 | 1,25 |
| P3 | 1,42 | *97,00* | 1,58 |
| P4 | 0,83 | 0,33 | *98,83* |
| P5 | <u>34,17</u> | 0,83 | <u>65,00</u> |
| P6 | 1,17 | *50,33* | *48,50* |

mean values (arithmetic)
**according to reference pattern , <u>deviated</u>**

|    | Ch1 | Ch2 | Ch3 |
|----|------|------|------|
| P1 | *5,25* | *6,12* | 5,17 |
| P2 | *4,69* | 2,45 | 3,25 |
| P3 | 2,75 | *5,02* | 3,34 |
| P4 | 3,51 | 1,55 | *4,81* |
| P5 | <u>13,61</u> | 2,26 | <u>14,07</u> |
| P6 | 3,51 | *6,48* | *7,29* |

threefold standard deviation
**according to reference pattern, <u>deviated</u>**

|    | Ch1 | Ch2 | Ch3 |
|----|------|------|------|
| P1 | 50 | 50 | 0 |
| P2 | 100 | 0 | 0 |
| P3 | 0 | 100 | 0 |
| P4 | 0 | 0 | 100 |
| P5 | 50 | 0 | 50 |
| P6 | 0 | 50 | 50 |

**NFI reference pattern**

|    | Ch1 | Ch2 | Ch3 |
|----|------|------|------|
| P1 | 10,00 | 10,00 | 9,17 |
| P2 | 10,00 | 1,67 | 6,25 |
| P3 | 7,08 | 10,00 | 7,92 |
| P4 | 4,17 | 1,67 | 10,00 |
| P5 | 10,00 | 4,17 | 10,00 |
| P6 | 5,83 | 10,00 | 10,00 |

**10% / 20%-off-set bands pertinent to 100% and 50%-mean values, and factor 5 pertinent to 0% values, i.e. accepted ranges for reference pattern**

## Simulated data set:

|    | Ch1 | Ch2 | Ch3 | Total |
|----|------|------|------|-------|
| P1 | 52 | 48 | 0 | 100 |
|    | 49 | 48 | 3 | 100 |
|    | 47 | 52 | 1 | 100 |
|    | 50 | 46 | 4 | 100 |
|    | 48 | 49 | 3 | 100 |
|    | 50 | 50 | 0 | 100 |
| P2 | 98 | 0 | 2 | 100 |
|    | 97,5 | 0 | 2,5 | 100 |
|    | 99 | 0 | 1 | 100 |
|    | 100 | 0 | 0 | 100 |
|    | 96 | 2 | 2 | 100 |
|    | 100 | 0 | 0 | 100 |
| P3 | 1 | 96 | 3 | 100 |
|    | 2 | 96 | 2 | 100 |
|    | 2 | 97 | 1 | 100 |
|    | 1 | 99 | 0 | 100 |
|    | 0 | 99 | 1 | 100 |
|    | 2,5 | 95 | 2,5 | 100 |
| P4 | 0 | 0 | 100 | 100 |
|    | 0 | 0 | 100 | 100 |
|    | 3 | 1 | 96 | 100 |
|    | 1 | 0 | 99 | 100 |
|    | 0 | 0 | 100 | 100 |
|    | 1 | 1 | 98 | 100 |
| P5 | 42 | 1 | 57 | 100 |
|    | 36 | 0 | 64 | 100 |
|    | 29 | 0 | 71 | 100 |
|    | 33 | 2 | 65 | 100 |
|    | 31 | 1 | 68 | 100 |
|    | 34 | 1 | 65 | 100 |
| P6 | 0 | 50 | 50 | 100 |
|    | 1 | 49 | 50 | 100 |
|    | 1 | 53 | 46 | 100 |
|    | 2 | 47 | 51 | 100 |
|    | 3 | 52 | 45 | 100 |
|    | 0 | 51 | 49 | 100 |

With regard to P5, a distribution of <<50 : ≈ 0 : »50 is found, with both mean values and scatter being outside the accepted ranges. The diagnosis "HBV-positive" is rejected and replaced by the alternative diagnosis "double infection". More specifically, the positive result is due to a co-infection with HIV-1-O and HCV as the most probable suggestion, assuming that the SFI ratio R1 / R3 is variable, and the final value is approximated late. For a co-infection of HCV and HBV, one would expect a more pronounced bias in favor of channel 3 (R3), and rapidly equalizing SFI ratios, considering the very high amplification/detection efficiency of the HBV assay. Therefore, HIV-1-O + HCV is the preferred diagnostic proposal.

**Claims**

1.   A method for the determination of at least 3 analytes comprising the steps:

a) Providing a mixture of a sample containing said at least 3 analytes or suspected of containing one or more of said analytes and at least 3 different binding partners under conditions allowing the specific binding of said binding partners to said analytes, wherein

- a first of said at least 3 binding partners is specific for a first of said at least 3 analytes and is coupled to a first label,

- a second of said at least 3 binding partners is specific for a second of said at least 3 analytes and is coupled to a second label, which label is separately detectable from the label coupled to said first binding partner, and

- a third of said at least 3 binding partners specific for a third of said at least 3 analytes whereby a first amount of said third binding partner is coupled to the same label as coupled to said first binding partner and a second amount of said third binding partner is coupled to the same label as the second binding partner and said first amount of said third binding partner is not coupled to the same label as coupled to the second binding partner and said second amount of said third binding partner is not coupled to the same label as coupled to the first binding partner,

b) Detecting the signal intensities indicative for said first and second label,

c) Determining the analytes present in said sample using said signal intensities detected in step b).

2.   Method of claim 1 **characterized in that** the different binding partners are nucleic acid sequence specific probes.

3.   Method of claim 2, **characterized in that** said analytes are amplified nucleic acids.

4.   Method of claim 3 **characterized in that** said nucleic acids were amplified using PCR.

5.   Method of any of claims 1 to 4 **characterized in that** said labels were detected homogeneously.

6.   Method of claim 5 **characterized in that** said labels were detected using fluorescence energy transfer.

7.   Method of any of claims 1 to 6 **characterized in that** a first of said first, second and third binding partners is specific for HIV, a second binding partner is specific for HCV and a third binding partner is specific for HBV.

8.   Method of any of claims 1 to 6 **characterized in that** a first binding partners is specific for HIV-1-M, a second binding partner is specific for HIV-1-O, a third binding partner is specific for HIV-2, a fourth binding partner is specific for HCV, a fifth binding partner is specific for HBV and a sixth binding partner is specific for HAV.

9.   A composition of matter comprising

- a first binding partner specific for a first analyte coupled to a first label,

- a second binding partner specific for a second analyte coupled to a second label, which label is separately detectable from the label coupled to said first binding partner, and

- a third binding partner specific for a third analyte whereby a first amount of said third binding partner is coupled to the same label as coupled to said first binding partner and a second amount of said third binding partner is coupled to the same label as the second binding partner and
said first amount of said third binding partner is not coupled to the same label as coupled to the second binding partner and said second amount of said third binding partner is not coupled to the same label as coupled to the first binding partner.

10. A kit for the determination of at least 3 analytes containing in one or more containers

- a first binding partner specific for a first analyte coupled to a first label,

- a second binding partner specific for a second analyte coupled to a second label, which label is separately detectable from the label coupled to said first binding partner, and

- a third binding partner specific for a third analyte, whereby a first amount of said third binding partner is coupled to the same label as coupled to said first binding partner and a second amount of said third binding partner coupled to the same label as said second binding partner.

11. Kit of claim 10 further containing means for specific binding of said binding partners to said analytes in said one container or in one or more further containers.

12. Kit of claim 10 or 11 further containing reagents for nucleic acid amplification in said one container or in one or more further containers.

13. Use of at least 3 binding partners for the determination of at least 3 analytes in a sample, wherein

- a first binding partner specific for a first analyte is coupled to a first label,

- a second binding partner specific for a second analyte is coupled to a second label, which label is separately detectable from the label coupled to said first binding partner, and

- a third binding partner specific for a third analyte, whereby a first amount of said third binding partner is coupled to the same label as coupled to said first binding partner and a second amount of said third binding partner is coupled to the same label as said second binding partner and
said first amount of said third binding partner is not coupled to the same label as coupled to the second binding partner and said second amount of said third binding partner is not coupled to the same label as coupled to the first binding partner.

Fig 1

| IC RESPONSE | / | TARGET |

............data collection as described above

IF S/N ≥ 1.13 in > 1 channel:

construction of a composite $AFI_{1+2 \text{ or } 1+3 \text{ or } 2+3 \text{ or } 1+2+3}$ curve
....$\{AFI = (TFI_{f(t)} - [DM * DD_{f(t)}] * XT)_i\}$ = arbitrary fluor. intensity

....AFI-over-time curves are fitted e.g. via third-grade polynomal

and its **slope profile** is established via 1. derivative using e.g. an update of SavGol V9 algorithm

significant
..... max. of ..........
1. deriv.
?
or
....S/N ≥ 1.5 ?

no → GO TO "xxx", **report "negative"**
End

yes

GO TO "yyy", **report "positive"** and **assign ct value**, AND

....calculate total $NFI_{1+2+3}$ = 100% target response intensity
(e.g. AUC, or plateau intensity)
$\{NFI = (AFI - BG) * NF_{Ri}\}$

....analyze its distribution across target reporter channels

compare to pre-set NFI distribution pattern and deduce type of infection, also taking into account end-point SFI ratios and SFI ratios as f(t), plus optionally absolute SFI signal levels and AFI/t curve shapes

pos.

**report "HXV infected"**

neg.

**flag, and order for retesting**

**European Patent Office**

**EUROPEAN SEARCH REPORT**

Application Number

EP 01 12 0466

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 759 781 A (BALLARD STEPHEN GWYNN ET AL) 2 June 1998 (1998-06-02) | 1,2,5, 9-11,13 | C12Q1/68 |
| Y | * column 2, line 1 - column 8, line 39; example 1 * | 3,4,6-8, 12 | |
| X | SPEICHER M R ET AL: "KARYOTYPING HUMAN CHROMOSOMES BY COMBINATORIAL MULTI-FLUOR FISH" NATURE GENETICS, NEW YORK, NY, US, vol. 12, April 1996 (1996-04), pages 368-375, XP000930084 ISSN: 1061-4036 | 1,2,5, 9-11,13 | |
| Y | * Methods * * page 368, column 1 - page 370, column 2; table 2 * | 3,4,6-8, 12 | |
| Y | VET J A M ET AL: "Multiplex detection of four pathogenic retroviruses using molecular beacons" PROCEEDINGS OF THE NATIONAL ACADEMY OF SCIENCES OF USA, NATIONAL ACADEMY OF SCIENCE. WASHINGTON, US, vol. 96, 1999, pages 6394-6399, XP002145609 ISSN: 0027-8424 * Abstract; Materials and Methods * | 6-8,12 | **TECHNICAL FIELDS SEARCHED (Int.Cl.7)** C12Q |
| Y | MERCIER BERNARD ET AL: "Simultaneous screening for HBV DNA and HCV RNA genomes in blood donations using a novel TaqMan PCR assay." JOURNAL OF VIROLOGICAL METHODS, vol. 77, no. 1, January 1999 (1999-01), pages 1-9, XP002193782 ISSN: 0166-0934 * whole document * | 6-8,12 | |

-/--

The present search report has been drawn up for all claims

| Place of search | Date of completion of the search | Examiner |
|---|---|---|
| THE HAGUE | 2 April 2002 | Aguilera, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 01 12 0466

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | TONG ANTHONY K ET AL: "Combinatorial fluorescence energy transfer tags for multiplex biological assays." NATURE BIOTECHNOLOGY, vol. 19, no. 8, August 2001 (2001-08), pages 756-759, XP002193783 August, 2001 ISSN: 1087-0156 * page 756, column 1, paragraph 2 - column 2, paragraph 1; figure 1 * | 6 | |
| A | MENG Q ET AL: "Automated multiplex assay system for simultaneous detection of hepatitis B virus DNA, hepatitis C virus RNA, and human immunodeficiency virus type 1 RNA." JOURNAL OF CLINICAL MICROBIOLOGY, vol. 39, no. 8, August 2001 (2001-08), pages 2937-2945, XP002193784 ISSN: 0095-1137 * page 2938, column 2, paragraph 1 * | 1-13 | |
| A,D | SAMIOTAKI M ET AL: "SEVEN-COLOR TIME-RESOLVED FLUORESCENCE HYBRIDIZATION ANALYSIS OF HUMAN PAPILLOMA VIRUS TYPES" ANALYTICAL BIOCHEMISTRY, ACADEMIC PRESS, SAN DIEGO, CA, US, vol. 253, no. 2, 15 November 1997 (1997-11-15), pages 156-161, XP000721248 ISSN: 0003-2697 * whole document * | 1-13 | |

TECHNICAL FIELDS SEARCHED (Int.Cl.7)

-/--

The present search report has been drawn up for all claims

| Place of search | Date of completion of the search | Examiner |
|---|---|---|
| THE HAGUE | 2 April 2002 | Aguilera, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

European Patent Office

## EUROPEAN SEARCH REPORT

Application Number

EP 01 12 0466

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | JOSEFSSON AGNETHA ET AL: "Detection and quantitation of human papillomavirus by using the fluorescent 5' exonuclease assay." JOURNAL OF CLINICAL MICROBIOLOGY, vol. 37, no. 3, March 1999 (1999-03), pages 490-496, XP002193785 ISSN: 0095-1137 * whole document * | 1-13 | |
| A,D | RIED T ET AL: "SIMULTANEOUS VISUALIZATION OF SEVEN DIFFERENT DNA PROBES BY IN SITU HYBRIDIZATION USING COMBINATORIAL FLUORESCENCE AND DIGITAL IMAGING MICROSCOPY" PROCEEDINGS OF THE NATIONAL ACADEMY OF SCIENCES OF USA, NATIONAL ACADEMY OF SCIENCE. WASHINGTON, US, vol. 89, 1 February 1992 (1992-02-01), pages 1388-1392, XP002073155 ISSN: 0027-8424 * whole document * | 1-13 | TECHNICAL FIELDS SEARCHED (Int.Cl.7) |

The present search report has been drawn up for all claims

| Place of search | Date of completion of the search | Examiner |
|---|---|---|
| THE HAGUE | 2 April 2002 | Aguilera, M |

**ANNEX TO THE EUROPEAN SEARCH REPORT
ON EUROPEAN PATENT APPLICATION NO.**

EP 01 12 0466

This annex lists the patent family members relating to the patent documents cited in the above-mentioned European search report.
The members are as contained in the European Patent Office EDP file on
The European Patent Office is in no way liable for these particulars which are merely given for the purpose of information.

02-04-2002

| Patent document cited in search report | | Publication date | Patent family member(s) | | Publication date |
|---|---|---|---|---|---|
| US 5759781 | A | 02-06-1998 | AU | 724912 B2 | 05-10-2000 |
| | | | AU | 1423597 A | 17-07-1997 |
| | | | CA | 2241148 A1 | 03-07-1997 |
| | | | EP | 0874912 A1 | 04-11-1998 |
| | | | JP | 2000507809 T | 27-06-2000 |
| | | | US | 6007994 A | 28-12-1999 |
| | | | WO | 9723648 A1 | 03-07-1997 |
| | | | CA | 2241364 A1 | 03-07-1997 |
| | | | EP | 0879297 A1 | 25-11-1998 |
| | | | JP | 11510707 T | 21-09-1999 |
| | | | WO | 9723649 A1 | 03-07-1997 |

EPO FORM P0459

For more details about this annex : see Official Journal of the European Patent Office, No. 12/82